# EUROPÄISCHE PATENTSCHRIFT

(11) **EP 1 583 566 B1**
(45) Veröffentlichungstag und Bekanntmachung des Hinweises auf die Patenterteilung: **20.02.2013**
(21) Anmeldenummer: 03795957.4
(22) Anmeldetag: 23.12.2003
(51) Int. Cl.: A61L 15/22, A61L 15/60, A61L 26/00, A61K 9/70

(54) **SELBSTKLEBENDE POLYMERMATRIX MIT EINEM GEHALT AN MEERESALGENEXTRAKT UND GLYCERIN**
SELF-ADHESIVE POLYMER MATRIX CONTAINING SEA ALGAE EXTRACT AND GLYCERIN
MATRICE POLYMERE AUTOADHESIVE CONTENANT DE L'EXTRAIT D'ALGUES MARINES ET DE LA GLYCERINE

(30) Priorität: 23.12.2002 DE 10260873
(43) Veröffentlichungstag der Anmeldung: 12.10.2005
(73) Patentinhaber: Beiersdorf AG, 20245 Hamburg (DE)
(72) Erfinder: WOELLER, Karl-Heinz, 20257 Hamburg (DE); BERG, Thorsten, 20149 Hamburg (DE)
(86) Internationale Anmeldenummer: PCT/EP2003/014793
(87) Internationale Veröffentlichungsnummer: WO 2004/058315

(56) Entgegenhaltungen:
- EP-A- 0 507 160
- EP-A- 0 970 707
- EP-A- 0 976 382
- EP-A- 1 097 712
- EP-A- 1 158 021

## Beschreibung

Die vorliegende Erfindung betrifft eine Verwendung von einem in Wasser gelbildendem Polymer, bevorzugt einem Polyacrylsäurepolymer, Wasser, einem mit Alkohol nicht gelbildendem Meeresalgenextrakt und einem ein- oder mehrwertigen Alkohol zur Einstellung der Klebrigkeit, Kohesivität, Konsistenz und Elastizität von selbstklebenden Polymermatrices. Die Matrix kann mit hydrophilen oder auch hydrophoben Wirkstoffen dotiert werden.

Der Wirkmechanismus von Pflastern zur Verabreichung pharmazeutischer Substanzen in die Haut unterliegt einem analogen Funktionsprinzip wie Transdermale Therapeutische Systeme (TTS).
Transdermale Therapeutische Systeme zur Abgabe von Wirkstoffen in bzw. durch die Haut sind seit langer Zeit bekannt und stellen pflasterartige, insbesondere arzneistoffdotierte Systeme dar.

Die topische Applikation von Arzneimitteln über wirkstoffhaltige Pflastersysteme bietet zwei Hauptvorteile:
● Erstens wird durch diese Darreichungsform eine Freisetzungskinetik des Wirkstoffes erster Ordnung realisiert, wodurch über einen sehr langen Zeitraum ein konstanter Wirkstoffspiegel im Organismus aufrechterhalten werden kann.
● Zweitens werden über den Aufnahmeweg durch die Haut der Magen-Darm-Trakt sowie die erste Leberpassage vermieden. Dadurch können ausgewählte Arzneistoffe in einer geringen Dosierung wirkungsvoll verabreicht werden. Dies ist insbesondere dann von Vorteil, wenn eine lokale Wirkung des Arzneistoffes unter Umgehung einer systemischen Wirkung erwünscht ist. Dies ist zum Beispiel bei der Behandlung rheumatischer Gelenkbeschwerden oder Muskelentzündungen der Fall.

Die zeitabhängige Freisetzung der Substanz, beispielsweise dem Arzneistoff, aus einem TTS erfolgt in Abhängigkeit ihres Verteilungskoeffizienten TTS/Haut und ihrer Diffusion im Bereich des TTS und der Haut.
Beide Faktoren werden durch die Zusammensetzung der Matrix bestimmt, wodurch die pro Zeiteinheit freigesetzte Menge und die Dauer der Wirksamkeit direkt beeinflusst werden können. Üblicherweise werden hierfür Hydrokolloide, Lösungsvermittler und Enhancer eingesetzt, welche eine verbesserte Löslichkeit und Diffusion sowie einen schnelleren Übergang der Substanz von TTS in die Haut ermöglichen.
Im Idealfall wird eine Freisetzungskinetik erster Ordnung erreicht, was eine Freisetzung gleicher Mengen pro Zeiteinheit ermöglicht.

Eine in der Fachliteratur gut beschriebene Ausführungsform solcher transdermalen Systeme stellen Matrixsysteme oder monolithische Systeme dar, in denen der Arzneistoff direkt in den druckempfindlichen Haftklebstoff eingearbeitet wird. Eine solche haftklebrige, wirkstoffhaltige Matrix ist üblicherweise im anwendungsfertigen Produkt auf der einen Seite mit einem für den Wirkstoff undurchlässigen Träger ausgestattet, auf der gegenüberliegenden Seite befindet sich eine mit einer Trennschicht ausgestatten Trägerfolie, die vor der Applikation auf die Haut entfernt wird (kleben&dichten, Nr.42, 1998, S. 26 bis 30).

Die vorgenannten Eigenschaften eines TTS vermeiden eine häufig zu wiederholende Applikation und Belastung der Haut mit hohen Konzentrationen an Wirkstoffen und verringern damit die Reizung der Haut, welche bei wiederholter Applikation von flüssigen und halbfesten Applikationsformen unumgänglich ist. Sollten dennoch im Verlauf einer TTS Applikation unerwünschte Wirkungen auftreten, kann durch das Entfernen des TTS die weitere Belastung sofort gestoppt werden.

Zusammengefasst liegen die Vorteile der TTS in einer deutlich verbesserten Compliance der Anwender, was auf die einfache und schnelle Applikation sowie die lange Wirksamkeit von Transdermalen Therapeutischen Systemen zurückzuführen ist.
Eine Grundanforderung an ein TTS ist einerseits ein gutes Haftvermögen auf Haut, das über den gesamten Zeitraum der beabsichtigten Wirkstoffdosierung aufrechterhalten bleiben muss, und andererseits eine rückstandsfreie Entfernbarkeit des TTS. Auch ein schmerzhaftes Wiederablösen des wirkstoffhaltigen Pflasters nach längerer Tragezeit wird häufig beobachtet. Neben Klebmassen, die in Lösung auf den Träger beschichtet werden, kommen u.a. auch lösungsmittelfreie Systeme, wie Heißschmelzklebmassen zum Einsatz. Diese zeichnen sich dadurch aus, dass bei der Beschichtung auf die Verwendung von organischen Lösungs- und Dispergiermittel verzichtet werden kann. Heißschmelzklebmassen werden durch Erwärmen in eine flüssige Form überführt und so als Schmelze auf den jeweiligen Pflasterträger aufgebracht. Neben technischen Gesichtspunkten, wie Lösungsmittelaufbereitung, Ausführungen der Anlagen mit Explosionsschutz und Umweltschutzauflagen, spielen auch medizinische Gründe für die Wahl von lösungsmittelfreien Klebstoffen eine Rolle. Transdermale therapeutische Systeme werden in der Regel auf gesunder, intakter Haut appliziert. Gerade hier ist es besonders wichtig, die Haut nicht durch ein Arzneimittel zu reizen oder gar zu schädigen. Eine häufig beobachtete Nebenwirkung ist dabei das Auftreten von Hautirritationen, die besonders bei längerer oder wiederholter Applikation eines TTS an einer gleichbleibenden Körperregion auftreten. Sie werden hauptsächlich durch die Inhaltsstoffe der haftklebrigen Matrix verursacht. Bei lösungsmittelhaltigen Systemen lässt sich nach dem Extrahieren der Lösungsmittel ein Restgehalt wiederfinden, der aufgrund des allergenen Potenzials ebenfalls zu unerwünschten Hautirritationen führen kann. Vor allem für die Anwendung auf Haut sind daher lösungsmittelfreie Systeme vorzuziehen, deren Formulierungen insbesondere hautfreundliche Inhaltstoffe beinhalten.

Selbstklebende Matrixsysteme zur Verabreichung von pharmazeutischen und/oder kosmetischen Wirkstoffen gehören in Asien, insbesondere in Japan, zu den traditionellen Anwendungen und werden im japanischen Arzneibuch unter dem Begriff "Cataplasma" definiert. Cataplasmen werden danach gewöhnlich durch Mischen von Glycerin, Wasser oder anderer geeigneter flüssiger Substanzen mit fein pulverisierten Wirkstoffen unter Zusatz essentiellen Ölen zubereitet.

Glycerin fungiert hierbei als Feuchthaltemittel, um ein vorzeitiges Austrocknen bei Anwendung der Cataplasmen zu verhindern.

Während in den traditionellen asiatischen Zubereitungen natürliche Verdickungsmittel wie Tonerde etc. zur Anwendung kommen, werden in den letzten Jahrzehnten mehr und mehr moderne synthetische Rohstoffe, wie z.B. Polyacrylsäure als Gelbildner, zur Herstellung eingesetzt. Dadurch lassen sich die gemeinhin pastösen Cataplasmen auch als Hydrogelmatrices mit verbesserter Anmutung und Anwenderfreundlichkeit darstellen. EP 1 136 057 A1 beschreibt ein wässriges Gelsystem zur kosmetischen Anwendung ohne Träger oder Abdeckung mit einer Lichtdurchlässigkeit von min. 70 %.
In EP 0 507 160 A1 werden Cataplasmen mit Lidocain enthaltend beschrieben.

Nachteilig an den beschriebenen Cataplasmen ist, dass zur Herstellung der Basismatrices viele verschiedene Einzelkomponenten wie Gelbildner, Verdicker, Weichmacher, Feuchthaltemittel, Stabilisatoren, Emulgatoren, pH-Regulatoren, Antioxidantien etc., bei wirkstoffhaltigen Cataplasmen evtl. noch zusätzlich Lösungsvermittler und Penetrationsbeschleuniger, benötigt werden. Da sich Klebverhalten und Konsistenz einer solchen Matrix aus dem Zusammenwirken aller Einzelkomponenten ergeben, gestaltet sich eine gezielte Produktentwicklung/- optimierung hinsichtlich dieser grundlegenden Produktanforderungen entsprechend zeitaufwändig und schwierig.

Die Herstellung von Polymermatrices, insbesondere Gelmatrices, aus Polyacrylaten ist ebenfalls seit vielen Jahren bekannt und wird z. B. in EP 0 507 160 A1, JP 11-228340 und JP 04178323 beschrieben. Gelmatrices werden u.a. als Klebgrundlage und Wirkstoffreservoir in transdermalen Systemen eingesetzt. Solche Systeme haben eine ausreichende Klebkraft, speziell auf feuchter Haut (Bukkalpflaster), lassen sich aufgrund ungenügender Kohäsivität bei Bedarf aber nicht vollständig wieder abziehen.
Polyacrylsäure muss zur Ausbildung eines Gels mit definierter Struktur vernetzt werden. Die Natur des Vernetzers trägt dabei entscheidend zur Struktur des resultierenden Gels bei. Die üblichen vernetzende Agenzien können dabei Metallionen (z.B.: Al³⁺-Ionen), oder organische Verbindungen sein. Die Vernetzung mit Aluminiumsalzen läuft über die Koordination der Sauerstofffunktionen der Polyacrylsäure an die Al³⁺-Ionen. Es bildet sich ein sehr engmaschiges Gel mit hoher Viskosität aus, wobei die Viskosität des Gels nur über die Menge an Vernetzer gesteuert werden kann (handbook of pressure sensitive adhesive technology, Seite 458 ff, 1999).
In JP 11-228340 werden Gele auf Polyacrylsäurebasis offenbart, die als Vernetzer Al⁺³ - Verbindungen nutzen. Der Einsatz der zwingend notwendigen Aluminiumverbindung als Vernetzungsagens ist begrenzt, da ansonsten die physikalischen Eigenschaften des Gels verschlechtert werden. Bei zu hohem Anteil an Aluminiumvernetzer wird das Gel zu hart.

Aus der Literatur sind weitere Beispiele der Vernetzung mit multivalenten Metallionen bekannt, z.B. US 3900610 (Zinksalze), US 3770780 oder US 3790533 (Titanverbindungen). Die ionische Vernetzung mit Metallionen führt zu harten, viskosen und wenig klebrigen Polymergelen (handbook of pressure sensitive adhesive technology, Seite 458 ff, 1999).

In EP 303 445 A1 wird ein Pflaster mit monolither Gelmatrix auf Basis wasserlöslicher Polymere offenbart. Als zwingend erforderliche Bestandteile sind Cleboprid oder ein pharmazeutisch akzeptables Salz davon als Wirkstoff, Wasser, wasseraufnehmende Agenzien und wasserlösliche Polymere vorgesehen. Als wasserlösliche Polymere kann der Fachmann aus einer Reihe bekannter Polymere wie Polyvinylalkohol, Gelatine, Polyacrylsäure, Natriumpolyacrylate, Methylcellulose, Carboxymethylcellulose, Polyvinylpyrrolidon, Gummi und anderen vernetzbaren Polymeren sowie Mischungen daraus auswählen

EP 976 382 A1 beschreibt ein Pflaster enthaltend eine Matrix, bestehend aus einem in wässriger Phase hydrophil gelierendes System, gebildet aus Gelangummi und mindestens einem weiteren Hydrokolloid. Beansprucht wird zwingend Gelangummi. Unter Gelangummi versteht der Fachmann, wie es Fachlexika definieren, Hydrokolloide, die aus folgenden Seepflanzen gewonnen werden: Agardhiella tenera, Furcellaria fastigiata, Hypnea cervicornis, musciformis, spicifera, Suhria vitata. Meeresalgenextrakte sind darunter nicht zu verstehen. Ebenso werden die wesentlichen Aspekte der selbstklebenden Eigenschaften, der Einstellbarkeit von Klebkraft und Elastizität der resultierenden Matrices nicht erwähnt.

Ein weiteres Problem bei der Vernetzung von Polyacrylsäure zu einer selbstklebenden Matrix bzw. Gel ist, dass eine einmal hergestellte Matrix mit definierten physikalischen Eigenschaften, Viskosität, Klebrigkeit etc. in einem späteren Herstellungsprozess die gleichen definierten Eigenschaften aufweisen muss. Diese Reproduzierbarkeit ist mit den derzeit bekannten Vernetzungstechnologien aufwändig oder gar nicht zu verwirklichen.

EP 1 158 021 A1 offenbart Mikrogele enthaltend viskositätserhöhende Verbindungen wie Acrylsäure-Alkylacrylat-Copolymere und hydrophile Verbindungen wie Agar oder Carrageenan.
EP 1 097 712 A1 offenbart Pflaster enthaltend Steroide, wobei die Basis für das gel Agar-Agar, Glycerin und Polyacrylsäure umfasst.
EP 970 707 A1 offenbart kühlende Gelpflaster umfassend Polyacrylat, Glycerin und Carrageenan sowie entzündungshemmende Wirkstoffe.

Aufgabe der vorliegenden Erfindung ist es daher ein einfaches Polymermatrixsystem für Cataplasmen/Hydrogele zu entwickeln, welches mit wenigen Einsatzstoffen gezielt Matrices mit bestimmter Konsistenz und Klebkraft herstellen lässt.
Weitere Aufgabe der vorliegenden Erfindung ist es eine Polymermatrix bereit zu stellen, in die wasserlösliche oder hydrophobe Wirkstoffe eingearbeitet werden können und diese gezielt an die Haut wieder abgegeben werden können.
Des weiteren ist eine Aufgabe der vorliegenden Erfindung Pflaster zur Verfügung zu stellen, die zuvor genannte Polymermatrices enthalten und als TTS verwendet werden können.

Gelöst werden diese Aufgaben durch die Verwendung entsprechend Anspruch 1 .

Es war überraschend und für den Fachmann außerordentlich erstaunlich, dass eine selbstklebende Polymermatrix aus einem in Wasser gelbildenden Polymer umfassend bevorzugt mindestens ein Polyacrylsäurepolymer, Wasser, Meeresalgenextrakt und Alkohol das Bündel an Aufgaben löst.

Die Matrix besteht aus einem aus einem in Wasser gelbildenden Polymer, bevorzugt Polyacrylsäuregel, als klebkraftbestimmender Komponente. Als Meeresalgenextrakt wird bevorzugt Agar-Agar eingesetzt. Als Alkohol wird insbesondere ein- oder mehrwertige Alkohole, bevorzugt Glycerin, eingesetzt, die als Konsistenzfaktoren wirken. Obwohl die Einzelkomponenten bekannterweise für die Herstellung von Cataplasmen oder Hydrogelen eingesetzt werden, war es bislang nicht bekannt Agar-Agar in Verbindung mit z.B. Glycerin gezielt als Konsistenzfaktoren für Polyacrylsäurematrices einzusetzen.

Durch eine Erhöhung des Anteils an Meeresalgenextrakt in Polymermatrices, wie Cataplasmen/Hydrogelen, wird die Festigkeit der Matrices erhöht. Dies erhöht jedoch auch die Steifigkeit und verringert die Klebrigkeit. Dieser Nachteil kann durch Zusatz von Alkohol, insbesondere von Glycerin, wieder ausgeglichen werden. Es kann somit eine gewünschte Elastizität der resultierenden Polymermatrix bei konstantem Anteil an Meeresalgenextrakt eingestellt werden.
Es zeigte sich demnach eine synergistische Kombination aus Meeresalgenextrakt und ein- oder mehrwertigen Alkoholen, bevorzugt Glycerin, um eine gewünschte Elastizität der Gelmatrices zu gewährleisten.
Grundlage für den Einsatz als Konsistenzfaktor ist, dass Meeresalgenextrakt, im Gegensatz zu insbesondere der weit verbreiteten Gelatine und anderen Konsistenzfaktoren, in Verbindung mit Alkoholen, wie z.B. Glycerin oder Propandiol, keine Gelbildung hervorruft. Da sich erfindungsgemäße ein- oder mehrwertige Alkohole, wie Glycerin oder Propandiol, in Wasser homogen verteilen, aber mit dem Meeresalgenextrakt keine Gele bilden, wirken solcher Art Alkohole somit als Elastizitätsfaktor für die Matrices.

Bevorzugt einzusetzender Meeresalgenextrakt ist neben Agar-Agar auch Carrageenan. Carrageenan ist ein hydrophiles Polysaccharid von hohem Molekulargewicht, das aus verschiedenen Rotalgen, vornehmlich Chondrus crispus, durch Heißwasserextraktion, nachfolgendem Ausfrieren und anschließender Reinigung gewonnen wird. Die Struktur von Carrageenan besteht hauptsächlich aus sich wiederholenden Galactose und 3,6 Anhydrogalactoseeinheiten, beide sowohl in sulfatierter wie unsulfatierter Form. Der wichtigste Unterschied zwischen kappa, iota und lambda Carrageenan ist die Anzahl und Position der Estersulfatgruppen an den sich wiederholenden Galactoseeinheiten.
Eine Gelbildung von Carrageenan ist nur in Gegenwart von Kationen möglich. Erfindungsgemäß bevorzugt sind kappa und iota Carrageenan, welche in Gegenwart von Calcium-(kappa und iota), Kalium- und Ammonium-Ionen (nur kappa) Gele bilden. Besonders vorteilhaft ist der Einsatz entsprechender Kationenhydroxide, da die zur Herstellung erfindungsgemäßer Gelmatrixsysteme ebenfalls eingesetzte Polyacrylsäure zur Ausbildung stabiler Gele neutralisiert werden muss.
Industriell angeboten wird Carrageenan z.B. von Lehmann & Voss & Co. unter den Bezeichnungen Gelcarin, Viscarin und Seaspen.

Meeresalgenextrakt, wie erfindungsgemäß besonders bevorzugt Agar-Agar, ist ein hydrophiles Kolloid von Polysaccharid-Struktur bestehend aus dem gelierenden Agarose und dem nichtgelierendem Agaropektin, das aus verschiedenen Meeresalgen der Rhodophyceen-Klasse durch Heißwasserextraktion, nachfolgendem Ausfrieren und anschließender Reinigung gewonnen wird. Industriell angeboten wird Agar-Agar z.B. von der Riedel de Haen AG.

Der Extrakt, insbesondere Agar-Agar oder Carrageenan, wird bevorzugt in einer Menge von 0,1 - 15 Gew.%, besonders bevorzugt zwischen 0,5 - 5 Gew.%, eingesetzt. Alle Prozentangaben beziehen sich dabei auf Gewichtsanteile der Polymermatrix sofern nicht Gegenteiliges angegeben ist.

Ein- oder mehrwertige Alkohole wie z.B. Glycerin (1,2,3-Propantriol), sind unter anderem als Lösungsvermittler oder Feuchthaltemittel weit verbreitet eingesetzte Hilfsstoffe der pharmazeutischen Industrie.
Ein- oder mehrwertigen Alkohole, wie z.B. Glycerin, werden erfindungsgemäß bevorzugt in einer Menge von 1 - 85 Gew.%, besonders bevorzugt zwischen 5 - 45 Gew.% eingesetzt.
Der Anteil an in Wasser gelbildendem Polymer wie z.B. Polyacrylsäuregel in der Matrix regelt das Haftvermögen. Im Gegensatz zu Agar-Agar bildet Polyacrylsäure aber sowohl mit Wasser wie auch mit Alkoholen Gele, so dass das durch den Anteil an Polyacrylsäure eingestellte Haftvermögen unabhängig vom jeweiligen Alkoholanteil konstant bleibt.

Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

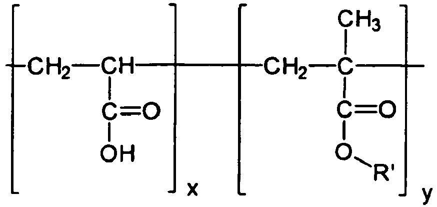

Darin stellen R' einen insbesondere langkettigen Alkylrest, und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind, bevorzugt Polyacrylate aus der Gruppe der Carbopole der Typen 980, 981, 1382, 2984, 5984 sowie besonders bevorzugt Carbomer 2001.
Ferner vorteilhaft sind Copolymere aus C₁₀₋₃₀-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Das in Wasser gelbildende Polymer, insbesondere Polyacrylsäure und/oder deren Copolymere, werden bevorzugt in einer Menge von 2 - 55 Gew.%, besonders bevorzugt zwischen 5 - 30 Gew.% eingesetzt.

Die Herstellung der Polymermatrices erfolgt ohne Verwendung organischer Lösemittel, vorzugsweise bei 40 - 95°C, in handelsüblichen Mischern/Knetern oder kontinuierlich in geeigneten Extrudern.
Als in Wasser gelbildendes Polymer eignet sich u.a. auch Affenbrotbaummehl.

Auf diese Weise lassen sich nur unter Verwendung von Wasser, in Wasser gelbildende Polymer, Meeresalgenextrakt und ein- oder mehrwertigem Alkohol als Ausgangsmaterialien gezielt weiche, geschmeidige, selbstklebende Hydrogelmatrices als Basis zur Herstellung und Anwendung als Pflaster, TTS, Cataplasmen oder Pads herstellen.

Zur Ausfertigung besonderer anwendungstechnischer Eigenschaften können die Polymermatrices mit entsprechenden Weichmachern, Lösungsvermittlern, Penetrationsenhancern, Neutralisationsmitteln wie z.B. Tromethamol (2-Amino-2-(hydroxymethyl)-1,3-propandiol), Triethanolamin (2,2',2"-Nitrilotriethanol) oder NaOH, Füllstoffen und/oder anderen bekannten Zusätzen versetzt werden, deren Zusatz jedoch nicht zwingend ist.

Die Gelmatrix kann somit mit hydrophilen, bei geeignetem Lösungsvermittler auch hydrophoben, Wirkstoffen zur Wundheilung oder Hautpflege dotiert werden. Bei der Einarbeitung hydrophober Wirkstoffe kann es von Nutzen sein, Cyclodextrine zur Verkapselung einzusetzen.

Cyclodextrine (Cycloamylosen, Cycloglucane) sind in kosmetischen und pharmazeutischen Zubereitungen an sich bekannt.
Die Verbesserung der Löslichkeit schwerlöslicher Substanzen in Gegenwart von Cyclodextrinen in wässrigem Milieu ist für einzelne Substanzen beschrieben. Vorteilhaft können sowohl die Einschlußverbindungen einer Substanz, auch Gast genannt, mit einer Cyclodextrinspezies, wobei sowohl 1:1 oder 1:2 Komplexe, wie auch Komplexe mit weiteren molaren Verhältnissen (Gast: Cyclodextrin) möglich sind, sowie auch deren physikalische Mischung sein.
Es handelt sich bei den Cyclodextrinen um zyklische Oligosaccharide bestehend aus α-1,4 verknüpften Glucosebausteinen. In der Regel sind sechs bis acht Glucosebausteine (α-, β-, bzw. γ-Cyclodextrin) miteinander verbunden.
Cyclodextrine werden bei Einwirkung von *Bacillus macerans* auf Stärke erhalten. Sie besitzen einen hydrophoben Innenraum und eine hydrophile Außenseite. Cyclodextrine und ihre Derivate können aufgrund Ihrer Struktur Inklusionskomplexe bilden. Sie sind zur "molekularen Verkapselung" von Wirkstoffen geeignet (z.B. als schützende Umhüllung empfindlicher Moleküle in kosmetischen und pharmazeutischen Formulierungen).
Diese Anwendungen sind auch in einer Reihe von Patenten beschrieben (z.B.:.WO 98/55148, EP 0 579 435, EP 0 392 608). In diesen Schriften wird jedoch meist nur ein Wirkstoff vom Cyclodextrin (-derivat) komplexiert. Mehrfachkomponenten-Inklusionskomplexe werden zwar in EP 0756 493 beschrieben, doch handelt es sich hier bei näherer Betrachtung um ein Salz und nicht um eine Zweikomponentenmischung von Säure und Base.

Mit "Cyclodextrin und/oder ein Derivat davon" sind im folgenden sowohl Cyclodextrine mit unterschiedlicher Anzahl von Glucosebausteinen im Ringmolekül als auch Derivate dieser Verbindungen gemeint.

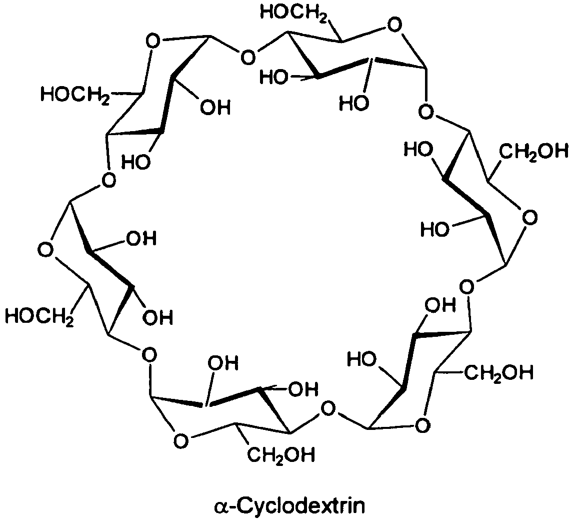

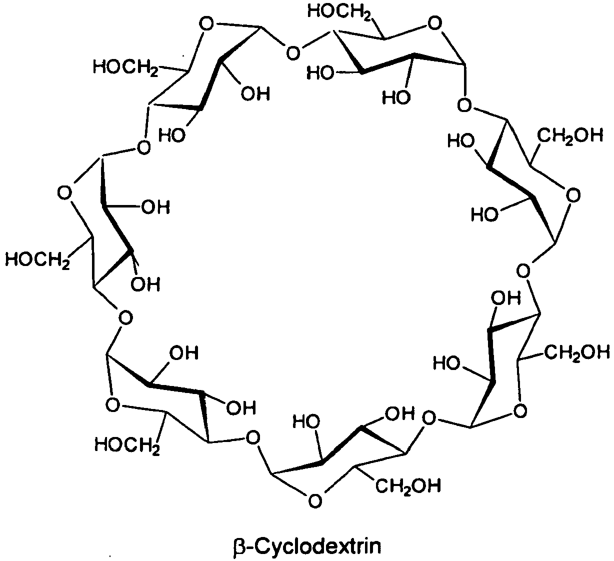

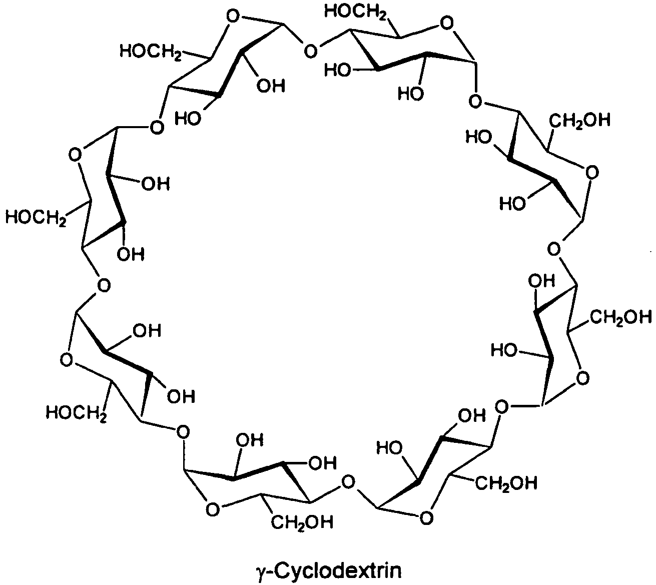

Das oder die Cyclodextrine werden bevorzugt in einer Konzentration von 0.0005 bis 20.0 Gewichts-%, insbesondere 0,01 bis 10 Gew.- % und besonders bevorzugt in einer Konzentration von 0.1 bis 5.0 Gew.%, eingesetzt.

Es ist erfindungsgemäß vorteilhaft native, polar- und/oder unpolar- substituierte Cyclodextrine einzusetzen. Hierzu gehören vorzugsweise aber nicht ausschließlich Methyl-, insbesondere random-Methyl-ß-Cyclodextrin, Ethyl- sowie Hydroxypropyl-Cyclodextrine, beispielsweise HP-β-Cyclodextrin oder HP-γ-Cyclodextrin.
Die erfindungsgemäß besonders bevorzugten Cyclodextrinspezies sind γ-Cyclodextrin sowie Hydroxypropyl-ß-Cylcodextrin.

Ein weiterer Stand der Technik ist in folgenden Schriften enthalten:
K. Uekama et al., Chemical Reviews, 1998, 98, 2045-2076, "Cyclodextrin drug carrier systems"
T. Loftsson, Int. J. Dermatology, 1998, 37, 241-246, "Cyclodextrins: new drugdelivery systems in dermatology".
J. Zatz et al. Cosmetics & Toiletries, 1997, 112, Juli, S. 39ff, "Applications of cyclodextrins in skin products.
U. Citernesi, Cosmetics & Toiletries, 1995, 110, März, S. 53 ff, Cyclodextrins in functional dermocosmetics.

Die erfindungsgemäß verwendeten Cyclodextrine bzw. Cyclodextrin-Gast-Inklusionskomplexe bzw. die Cyclodextrin-Substanz Mischungen lassen sich ohne Schwierigkeiten in die Polymermatrix einarbeiten.

In einer erfindungsgemäß besonders bevorzugten Ausführungsform enthält die Polymermatrix bzw. Gelmatrix pharmazeutische Wirkstoffe zur kontrollierten lokalen bzw. systemischen Abgabe an/in die Haut, in Mengen von 0 - 35 Gew.%, bevorzugt 0 - 15 Gew.%.

Als Wirkstoffe können beispielsweise ätherische Öle eingesetzt werden. Unter ätherischen Ölen sind aus Pflanzen gewonnene Konzentrate zu verstehen, die als natürliche Rohstoffe hauptsächlich in der Parfüm- und Lebensmittelindustrie eingesetzt werden und die mehr oder weniger aus flüchtigen Verbindungen bestehen. Als Beispiele für diese Verbindungen können 1,8-Cineol, Limonen, Menthol, Borneol und Kampfer genannt werden. Oft wird der Begriff ätherische Öle für die noch in den Pflanzen enthaltenen flüchtigen Inhaltsstoffe verwendet. Im eigentlichen Sinn versteht man aber unter ätherischen Ölen Gemische aus flüchtigen Komponenten, die durch Wasserdampfdestillation aus pflanzlichen Rohstoffen hergestellt werden.
Ätherische Öle bestehen ausschließlich aus flüchtigen Komponenten, deren Siedepunkte in der Regel zwischen 150 und 300 °C liegen. Sie enthalten überwiegend Kohlenwasserstoffe oder monofunktionelle Verbindungen wie Aldehyde, Alkohole, Ester, Ether und Ketone. Stammverbindungen sind Mono- und Sesquiterpene, Phenylpropan-Derivate und längerkettige aliphatische Verbindungen.
Bei manchen ätherischen Öle dominiert ein inhaltsstoff (zum Beispiel Eugenol in Nelkenöl mit mehr als 85%), andere ätherische Öle stellen hingegen komplex zusammengesetzte Mischungen der einzelnen Bestandteile dar. Oft werden die organoleptische Eigenschaften nicht von den Hauptkomponenten, sondern von Neben- oder Spurenbestandteilen geprägt, wie zum Beispiel von den 1,3,5-Undecatrienen und Pyrazinen im Galbanum-Öl. Bei vielen der kommerziell bedeutenden ätherischen Öle geht die Zahl der identifizierten Komponenten in die Hunderte. Sehr viele inhaltsstoffe sind chiral, wobei sehr oft ein Enantiomer überwiegt oder ausschließlich vorhanden ist, wie zum Beispiel (-)-Menthol im Pfefferminzöl oder (-)-Linalylacetat im Lavendelöl.

Als bevorzugte ätherische Öle können Oleum Eucalypti, Oleum Menthae piperitae, Oleum camphoratum, Oleum Rosmarini, Oleum Thymi, Oleum Pini sibricum und Oleum Pini silverstris sowie die Terpene 1,8-Cineol und Levomethanol genannt werden.

Als weitere ätherische Öle sind Oleum Abietis albae, Oleum Anisi, Oleum Aurantii Floris, Oleum Bergamottae, Oleum Calendulae infusum, Oleum camphoratum, Oleum Caryophylli, Oleum Chamomillae, Oleum Cinnamomi ceylanici, Oleum Citri, Oleum Citronellae, Oleum Cupressi, Oleum Cymbopogonis, Oleum Jecoris, Oleum Lavendulae, Oleum Macidis, Oleum Majoranae, Oleum Melaleucae viridiflorae, Oleum Melissae, Oleum Menthae arvensis, Oleum Menthae piperatae, Oleum Millefolium, Oleum Myrrhae, Oleum Myrte, Oleum Oregani, Oleum Pini sibricum, Oleum Pinisilvestris, Oleum Salviae, Oleum Santali, Oleum Terebinthinae rectificat., Oleum Thymi Oleum Valerianae, Oleum Zingiberis und/oder Teebaumöl zu nennen.

Pfefferminzöle sind durch Wasserdampfdestillation aus Blättern und Blütenständen verschiedener Pfefferminze-Sorten gewonnene ätherische Öle, gelegentlich auch solche aus Mentha arvensis.

Citrusöle sind ätherische Öle, die aus den Schalen von Citrusfrüchten (Bergamotte, Grapefruit, Limette, Mandarine, Orange, Zitrone) gewonnen werden, oft auch Agrumenöle genannt.
Citrusöle bestehen zu einem großen Teil aus Monoterpen-Kohlenwasserstoffen, hauptsächlich Limonen (Ausnahme: Bergamottöl, das nur ca. 40% enthält).

Beispielsweise kann Menthol zur Oberflächenanästhesierung bei Hautirritationen durch leichte Verbrennungen eingesetzt werden. Die so hergestellten Produkte erzeugen ein angenehmes Kältegefühl und können zur Kühlung von kleineren Verbrennungen, die keiner fachärztlichen Behandlung bedürfen, zum Einsatz kommen.

Menthol hat drei asymmetrische C-Atome und kommt demzufolge in vier diastereomeren Enantiomerenpaaren vor (vgl. die Formelbilder, die anderen vier Enantiomeren sind die entsprechenden Spiegelbilder).

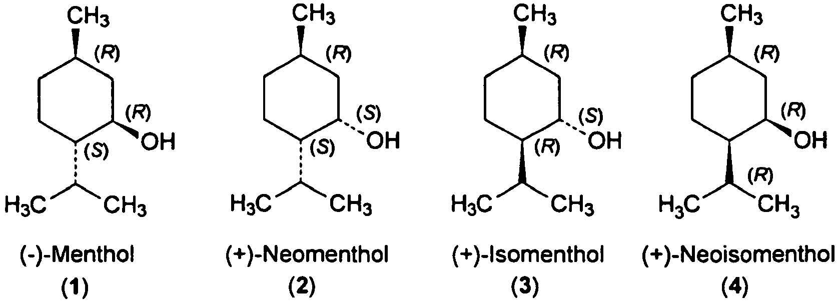

Die Diastereomeren, die destillativ getrennt werden können, werden als Neoisomenthol, Isomenthol, Neomenthol [(+)-Form: Bestandteil des japanischen Pfefferminzöls] und Menthol bezeichnet. Wichtigstes Isomer ist (-)-Menthol (Levomenthol), glänzende, stark pfefferminzartig riechende Prismen.

Als weitere Wirkstoffe kann zum Beispiel Campher zur Behandlung von rheumatischen Schmerzen, Neuralgien und Entzündungen der Matrix zugesetzt werden. Unter Campher versteht man 2-Bornanon, 1,7,7-Trimethylbicyclo[2.2.1]heptan-2-on, siehe untere Abbildung.

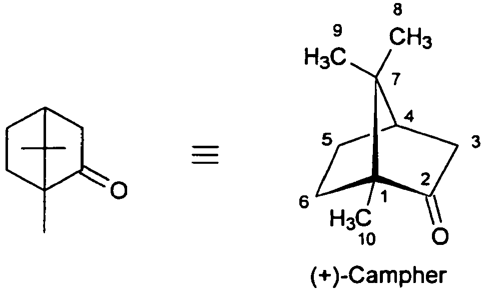

Aber auch in Kombination mit pflegenden Substanzen wie Jojobaöl oder Aloe vera ist die erfindungsgemäße Polymermatrix zu verwenden. Solche Kombinationen können je nach Anwendungsdefinition aus einem Arzneimittel ein Kosmetikum machen und somit die Zeit bis zur Vermarktung aufgrund der Verminderung der Zulassungszeiten drastisch verkürzen.

Daneben können für vorteilhafte Ausführungsformen der Hydrogele/Cataplasmen auch hyperämisierende Wirkstoffe wie natürliche Wirkstoffe des Cayenne-Pfeffers oder synthetische Wirkstoffe wie Nonivamid, Nicotinsäurederivate, bevorzugt Bencylnicotinat oder Propylnicotinat, genannt werden beziehungsweise Antiphlogistika und/oder Analgetika.

### Beispielhaft seien Capsaicin

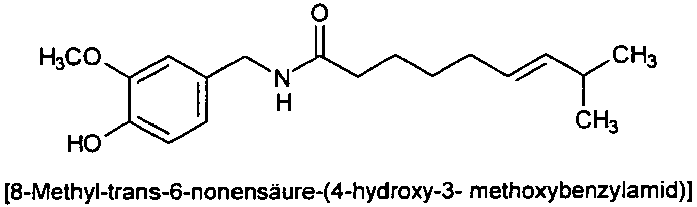

### Nonivamid

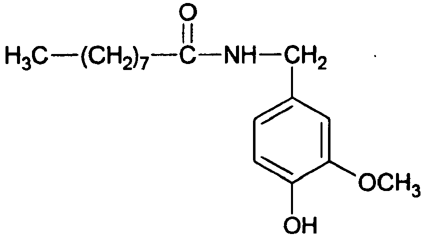

### Nicotinsäurebenzylester

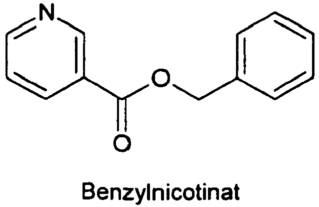

genannt.

Auch Flavon und seine Derivate (oft auch kollektiv "Flavone" genannt) sind vorteilhafte Zusatzstoffe im Sinne der vorliegenden Erfindung. Sie sind durch folgende Grundstruktur gekennzeichnet (Substitutionspositionen angegeben):

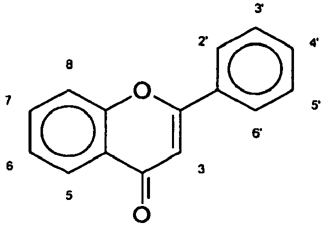

Einige der wichtigeren Flavone, welche auch bevorzugt in erfindungsgemäßen Zubereitungen eingesetzt werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kampferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt aus der Gruppe der Substanzen der generischen Strukturformel 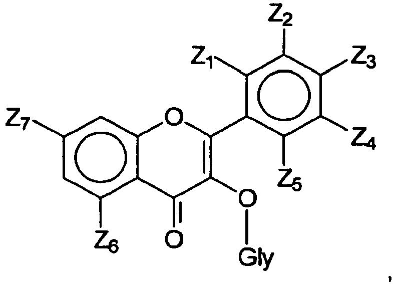 wobei Z₁ bis Z₇ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfingdungsgemäß können die Flavonoide aber auch vorteilhaft gewählt werden aus der Gruppe der Substanzen der generischen Strukturformel 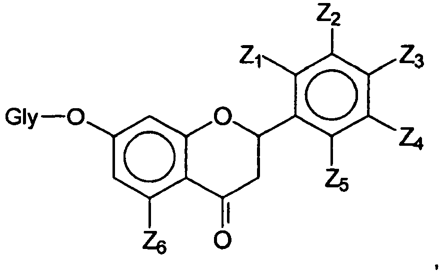 wobei Z₁ bis Z₆ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 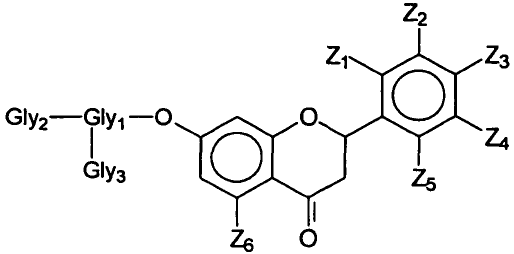 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur:

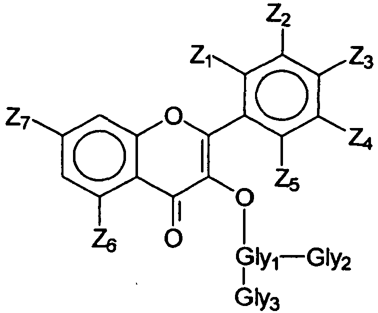

Besonders vorteilhaft werden die erfindungsgemäßen Flavonglycoside aus der Gruppe, welche durch die folgende Struktur wiedergegeben werden: 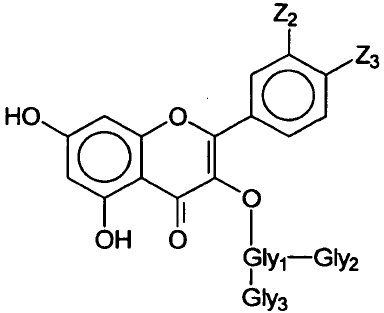 wobei Gly₁, Gly₂ und Gly₃ unabhängig voneinander Monoglycosidreste oder Oligoglycosidreste darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-gluco-pyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomareïn (3',4',7,8-Tetrahydroxyflava-non-7-glucosid) und isoquercetin (3,3',4',5,7-Pentahydroxyflavanon-3-(β-D-Glucopyranosid).

Weitere bevorzugte pharmazeutische Wirkstoffklassen einer Gelmatrix sind - ohne den Anspruch der Vollständigkeit im Rahmen der vorliegenden Erfindung zu erheben:
Antimykotika, wie z.B. Nafitin, Amorrolfin, Tolnaftat, Ciclopirox
Nichtsteroidale Antirheumatika, wie z.B. Glykolsalicylat, Flufenaminsäure, ibuprofen, Etofenamat, Ketoprofen, Piroxicam, Indomethacin
Antipuriginosa, wie z.B. Polidocanol, Isoprenalin, Crotamiton
Lokalanästhetika, wie z.B. Lidocain, Benzocain
Antipsoriatika, wie z.B. Ammoniumbitumasulfonat
Keratolytika, wie z.B. Harnstoff

Von besonderer Bedeutung unter den Wirkstoffen sind für Polymermatrices, Hydrogele/Cataplasmen oder Pads die Desinfektionsmittel beziehungsweise Antiseptika hervorzuheben.

Als Desinfektionsmittel werden Stoffe bezeichnet, die zur Desinfektion, d. h., zur Bekämpfung pathogener Mikroorganismen, zum Beispiel Bakterien, Viren, Sporen, Klein- und Schimmelpilze, geeignet sind. Im allgemeinen werden die Mittel an der Oberfläche von Haut, Kleidung, Geräten, Räumen, aber auch von Trinkwasser, Nahrungsmitteln, Saatgut (Beizen) und als Bodendesinfektionsmittel angewendet.

Besonders lokal anzuwendende Desinfektionsmittel, zum Beispiel zur Wunddesinfektion, werden auch als Antiseptika bezeichnet.

Desinfektionsmittel werden definiert als Stoffe oder Stoffgemische, die bei der Anwendung auf Gegenständen oder Oberflächen diese in einen Zustand versetzen, dass sie keine Infektion mehr verursachen. Ihre Wirkung muss bakterizid, fungizid, viruzid und sporizid, d.h. der Sammelbegriff: mikrobizid, sein. Ein Effekt im Sinne der Bakteriostase ist für Desinfektionsmittel unzureichend. Sie sind daher im allgemeinen pantoxisch, d. h. sie entfalten ihre Wirkung gegen alle lebenden Zellen.

Je nach Verwendungszweck teilt man die Desinfektionsmittel ein in solche zur Wäsche-, Flächen-, Instrumenten-, Haut- und Hände- sowie zur Stuhl- und Sputumdesinfektion. Unter Desinfektionsreiniger versteht man solche Desinfektionsmittel, die auch als Reinigungs- und gegebenenfalls Pflegemittel fungieren.

Unter Berücksichtigung der vielfältigen Forderungen, die an Desinfektionsmittel gestellt werden, wie zum Beispiel breites Wirkungsspektrum, kurze Einwirkungszeiten, Hautverträglichkeit, geringe Toxizität, Materialverträglichkeit usw. kommen nur einige Wirkstoff-Typen für den gewünschten Einsatz in Betracht.
1. Die wichtigste Wirkstoff-Gruppe sind die Aldehyde (Formaldehyd, Glyoxal, Glutaraldehyd). Sie besitzen ein breites Wirkungsspektrum einschließlich Virus-Wirksamkeit und sporizider Wirkung bei Formaldehyd und Glutaraldehyd.
2. Phenol-Derivate besitzen eine gute bakterizide Wirkung, sind aber nicht sporizid. Gegenüber fast allen anderen Desinfektionsmittelwirkstoffen haben sie den Vorzug, durch Schmutz verhältnismäßig wenig beeinflusst zu werden. Sie eignen sich daher bes. zur Stuhldesinfektion. Typische Vertreter sind 2-Biphenylol und p-Chlor-m-kresol (4-Chlor-3-methylphenol).
3. Alkohole zeichnen sich durch schnelle Wirksamkeit aus, allerdings erst bei relativ hohen Konzentrationen von ca. 40-80%.
4. Die quaternären Ammonium-Verbindungen, Kationentenside (Invertseifen) und Amphotenside gehören zur Klasse der Tenside. Sie zeichnen sich durch recht gute Haut- und Materialverträglichkeit sowie Geruchsneutralität aus. Ihr Wirkungsspektrum ist dagegen nur begrenzt. Hierher gehören zum Beispiel Benzalkoniumchlorid, Cetrimoniumbromid, Cetylpyridiniumchlorid (Hexadecylpyridiniumchlorid) und andere.

Quaternäre Ammoniumverbindungen sind organisch Ammoniumverbindungen mit quaternären Stickstoffatomen. Quaternäre Ammoniumverbindungen mit einem hydrophoben Alkyl-Rest sind biozid; ihr Einsatz ist freilich aus toxikologischen Gründen rückläufig.

Quaternäre Ammoniumverbindungen werden durch Umsetzung tertiärer Amine mit Alkylierungsmitteln, wie zum Beispiel Methylchlorid, Benzylchlorid, Dimethylsulfat, Dodecylbromid, aber auch Ethylenoxid hergestellt. In Abhängigkeit von dem eingesetzten tert. Amin unterscheidet man drei Gruppen: 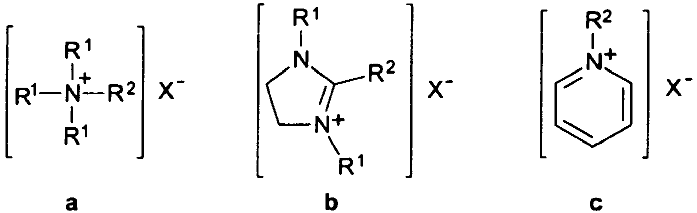
a) Lineare Alkylammoniumverbindungen
b) Imidazoliniumverbindungen
c) Pyridinium-Verb. R¹ = CH₃, R² = C₈₋₁₈, X = Halogen.

Die Alkylierung tertiärer Amine mit einem langen Alkylrest und zwei Methylgruppen gelingt besonders leicht, auch die Quaternierung tertiärer Amine mit zwei langen Resten und einer Methylgruppe kann mit Hilfe von Methylchlorid unter milden Bedingungen durchgeführt werden. Amine, die über drei lange Alkylreste oder hydroxysubstituierte Alkylreste verfügen, sind wenig reaktiv und werden bevorzugt mit Dimethylsulfat quaterniert.
5. Von den Halogenen besitzen Chlor und lod eine gewisse Bedeutung als Desinfektionsmittel. Chlor ist von der Wasseraufbereitung und Schwimmbaddesinfizierung her bekannt und damit seine unangenehmen Eigenschaften wie Geruch und Korrosivität. Trotz der ausgezeichneten Wirkung gegen Bakterien, Pilze, Sporen und Viren haben chlorhaltige Desinfektionsmittel im Humanbereich aus den obengenannten Gründen und wegen der starken Chlor-Zehrung durch organ. Substanzen keine starke Verbreitung gefunden. Dagegen werden Hypochlorite, Chlorkalk- und Chlorisocyanursäuren als technische Desinfektionsmittel noch umfänglich benutzt. Iodtinktur wird im medizinischen Bereich als Antiseptikum verwendet.
6. Desinfektionsmittel auf Basis von aktivem Sauerstoff (zum Beispiel Wasserstoffperoxid, Peroxyessigsäure) haben in letzter Zeit wieder etwas an Bedeutung gewonnen.
7. Silber wirkt, auch in gebundener Form, stark antiseptisch, da die in der Oxid-Schicht der Metalloberfläche enthaltenen Ag-Ionen in den Mikroorganismen eine blockierende Wirkung auf die Thiol-Enzyme ausüben. Ag-Ionen wirken auch stark fungizid und bakterizid. Dünne, bakterientötende Silber-Folien werden deshalb als Wundverbandmaterial verwendet, desgleichen Silber-Aerosole, Silber-Lösungen, Silberhaltige Salben, Tabletten und dgl. als Antiseptika und Antimykotika.

Die Silber-Ionen können dabei in Form von Salzen, Zeolithen, z.B. Aluminiumsilikate, oder bevorzugt Silbergläsern eingesetzt werden.

Außer den genannten Mikrobizid-Wirkstoffen sind noch eine Anzahl von mikrobistat. Substanzen und Konservierungsmitteln (Diphenylether, Carbanilide, Acetanilide aromatischen Säuren und deren Salze) für spezifische Verwendung auf dem Markt, die im erweiterten Sinne den Desinfektionsmitteln zugerechnet werden.

Eine einheitliche Wirkungsweise der Desinfektionsmittel ist nicht zu erkennen. Während manche Präparate auf die Cytoplasmamembran der Bakterien zerstörend wirken sollen, wird von anderen eine irreversible Blockierung wichtiger Sulfidbindungen bei Enzymen oder von Spurenelementen durch Chelatisierung angenommen.

Gegenstand der Erfindung ist dementsprechend auch die Verwendung von desinfizierenden Mittel in Polymermatrices, welche
mindestens ein nichtionisches Tensid und
mindestens eine Aminosäure und/oder ein Aminosäurenderivat
sowie mindestens ein desinfizierendes Agens und/oder einen mikrobiziden Wirkstoff enthalten.

Vorteilhaft werden das oder die nichtionische Tenside gewählt aus der Gruppe der Alkylethoxylate und/oder Alkylpropxylate, deren Alkylgruppe eine gesättigte oder ungesättigte, gerad- oder verzweigtkettige Alkylgruppe mit (8) 10 bis 18, vorzugsweise 12 bis 14 Kohlenstoffatomen ist, wobei sie vorzugsweise pro Molekül 2 bis 15, insbesondere 5 bis 9, speziell 7 Ethylenoxideinheiten enthalten. Ganz besonders bevorzugt sind Isotridecanolethoxylat und/oder Fettalkoholpolyglykolether.

Vorteilhaft wird die Gesamtmenge an nichtionischen Tensiden (eine oder mehrere Verbindungen) aus dem Bereich von 1,0 bis 20,0 Gew.-%, vorzugsweise von 5,0 bis 15,0 Gew.-% gewählt, jeweils bezogen auf das Gesamtgewicht der Matrix.

Vorteilhafte Aminosäuren sind zum Beispiel die Glutaminsäure, welche sich durch die folgende Strukturformel auszeichnet: 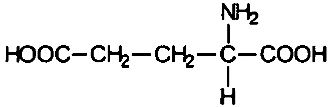 und/oder die Pyrrolidoncarbonsäure (Pyroglutaminsäure), welche sich durch die folgende Strukturformel auszeichnet:

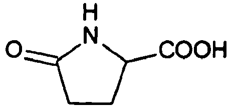

Vorteilhaft wird die Gesamtmenge an Aminosäuren (eine oder mehrere Verbindungen) aus dem Bereich von 0,1 bis 10,0 Gew.-%, vorzugsweise von 0,5 bis 2,0 Gew.-% gewählt, jeweils bezogen auf das Gesamtgewicht der Matrix.

Das oder die desinfizierenden Agenzien (mikrobiziden Wirkstoffe) werden bevorzugt gewählt aus der Gruppe der Aldehyde (zum Beispiel Formaldehyd, Glyoxal, Glutaraldehyd), der Phenol-Derivate (zum Beispiel 2-Biphenylol und p-Chlor-m-kresol (4-Chlor-3-methylphenol), der Alkohole, der quaternären Ammonium-Verbindungen (zum Beispiel Benzalkoniumchlorid, Cetrimoniumbromid, Cetylpyridiniumchlorid (Hexadecylpyridiniumchlorid). Aldehyde und quaternäre Ammoniumverbindungen sind dabei ganz besonders bevorzugt.

In einer besonders vorteilhaften Ausführungsform können die desinfizierenden Systeme ferner Amphotenside enthalten. Amphotenside sind Tenside, die sowohl saure als auch basische hydrophile Gruppen besitzen und sich also je nach Bedingung sauer oder basisch verhalten. Vorteilhaft sind beispielsweise Amphotenside auf der Basis von aliphatischen Polyaminen mit Carboxy-, Sulfo- oder Phosphono-Seitenketten, wie beispielsweise R-NH-(CH₂)ₙ-COOH.

Bevorzugt sind zum Beispiel Amphotenside, deren Alkylgruppe eine gesättigte oder ungesättigte, gerad- oder verzweigtkettige Alkylgruppe mit 10 bis 18, vorzugsweise 12 bis 14 Kohlenstoffatomen ist.

Insbesondere vorteilhaft sind ferner Amphotenside aus der Gruppe der Amphopropionate, wie zum Beispiel das Cocobetainamido Amphopropionat, welches sich durch die folgende Struktur auszeichnet:

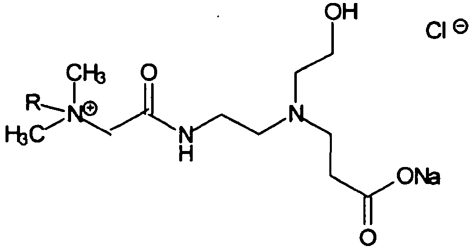

Vorteilhaft wird die Gesamtmenge an Amphotensiden (eine oder mehrere Verbindungen) aus dem Bereich von 1,0 bis 10,0 Gew.-%, vorzugsweise von 2,0 bis 5,0 Gew.-% gewählt, jeweils bezogen auf das Gesamtgewicht der Matrix.

Vorteilhaft ist es, die Verdünnung so durchzuführen, dass der Gehalt der einzelnen Substanzen in der Gebrauchslösung wie folgt ist:

| | |
|---|---|
| nichtionische Tenside: | zwischen 0,005 und 1 Gew.-% |
| Aminosäure: | zwischen 0,0005 und 0,5 Gew.-% |
| gegebenenfalls Amphotenside: | zwischen 0,005 und 0,5 Gew.-% |
| desinfizierende Agenzien: | zwischen 0,01 und 2,0 Gew.-% |

Zusätzlich zu den vorstehend genannten Komponenten können die desinfizierenden Systeme für derartige Zubereitungen übliche Konservierungsstoffe, Farbstoffe, Duftstoffe und/oder andere übliche Hilfsstoffe enthalten. Es ist jedoch auch möglich, solche Komponenten zu verwenden, die eine (konservierende, pflegende usw.) Wirkung entfalten und dabei gleichzeitig für eine bestimmte Farbe und/oder einen angenehmen Duft sorgen.

Die jeweils einzusetzenden Mengen an derartigen Trägerstoffen und Parfüm können in Abhängigkeit von der Art des jeweiligen Produktes vom Fachmann durch einfaches Ausprobieren leicht ermittelt werden.

Vorteilhaft ist auch die Verwendung von desinfizierenden Systemen, welche mindestens einen mikrobiziden Wirkstoff, gewählt aus der Gruppe der Alkylamine mindestens eine Aminosäure und/oder ein Aminosäurenderivat mindestens eine quaternäre Ammoniumverbindung enthalten.

Vorteilhaft werden die quaternären Ammonium-Verbindungen bevorzugt gewählt aus der Gruppe Benzalkoniumchlorid, Didecyldimethylammoniumchlorid, Cetrimoniumbromid, Cetylpyridiniumchlorid (Hexadecylpyridiniumchlorid). Vorteilhaft ist das Alkylamin das Dodecylbispropylentriamin.

Erfindungsgemäß vorteilhaft werden zusätzlich nichtionische Tenside zugesetzt, insbesondere vorteilhaft gewählt aus der Gruppe der Alkylethoxylate, deren Alkylgruppe eine gesättigte oder ungesättigte, gerad- oder verzweigtkettige Alkylgruppe mit 8 bis 18, vorzugsweise 12 bis 14 Kohlenstoffatomen ist, wobei sie vorzugsweise pro Molekül 2 bis 15, insbesondere 5 bis 9, speziell 7 Ethylenoxideinheiten enthalten. Ganz besonders bevorzugt sind Isotridecanolethoxylat und/oder Fettalkoholpolyglykolether.

Vorteilhaft wird die Gesamtmenge an nichtionischen Tensiden (eine oder mehrere Verbindungen) aus dem Bereich von 1,0 bis 20,0 Gew.-%, vorzugsweise von 5,0 bis 15,0 Gew.-% gewählt, jeweils bezogen auf das Gesamtgewicht der Matrix.

Weiterhin sind als Mittel zur Desinfektion, Konservierung und Antiseptik eine Vielzahl mikrobizid wirksamer chemischer Substanzen beziehungsweise Gemische dieser Substanzen an sich bekannt. Mikrobizide Substanzen sind im allgemeinen gegen das übliche Spektrum von Keimen, wie beispielsweise grampositive Bakterien, gramnegative Bakterien, Mykobakterien, Hefen, Pilze, Viren und dergleichen, mehr oder weniger wirksam, so dass man üblicherweise eine ausreichende Desinfektion, Konservierung oder Antiseptik durch geeignete Wirkstoffkombinationen erzielen kann.

Man kennt zur Desinfektion, Konservierung und Antiseptik eine Reihe von Wirkstoffen, insbesondere Aldehyde, wie beispielsweise Formaldehyd oder Glutaraldehyd, quaternäre Ammoniumverbindungen und langkettige Amine, Phenole oder Alkohole.

Aldehyde fixieren Reste von Blut und Eiweiß durch chemische Reaktion an den zu desinfizierenden Gegenständen, so dass diese nach der Desinfektion schwer zu reinigen sind. Außerdem haben sie ein vergleichsweise hohes allergenes Potential, so dass Anwendungen auf Haut und Händen nur in geringen Konzentrationen möglich sind oder aber in Kombination mit weiteren Wirkstoffen in Betracht kommen, um die erforderliche Unterschreitung der Sensibilisierungsschwelle einhalten zu können. Höhere Konzentrationen von Aldehyden sind auch wegen ihrer Geruches unerwünscht, so dass man auch aus diesem Grund die Konzentration durch Kombination mit weiteren Wirkstoffen verringert.

Quaternäre Ammoniumverbindungen und langkettige Amine werden häufig in der Flächendesinfektion und zur manuellen Instrumentendesinfektion sowie in geringem Umfang auch in der Händeantiseptik verwendet. Im Vergleich zu den Aldehyden ist der Geruch dieser Verbindungen deutlich weniger unangenehm. Eine chemische Reaktion mit Eiweißen erfolgt nicht, jedoch kommt es zu einer physikalischen Fällung von Eiweißen, die zum Teil durch geschickte Kombination mit Tensiden kompensiert werden kann. Für die maschinelle Instrumentendesinfektion sind die quaternären Ammoniumverbindungen nicht geeignet, weil es infolge der Turbulenzen in der Reinigungsmaschine zu einer starken, unerwünschten Schaumbildung kommt. Bei der Flächendesinfektion zeigen quaternäre Ammoniumverbindungen eine starke Tendenz, auf den Oberflächen "aufzuziehen", d. h., es bilden sich Schichten dieser Verbindungen auf den Oberflächen aus. Ein weiterer entscheidender Nachteil ist das eingeengte Wirkungsspektrum quaternärer Ammoniumverbindungen, da diese weder sporizid noch gegen unbehüllte Viren wirken.

Phenole sind vor allem wegen ihres Geruches, ihrer geringen Wirksamkeit gegen den Poliovirus, ihrer zum Teil schlechten Abbaubarkeit, ihrer hohen Lipidlöslichkeit verbunden mit einer starken Penetration durch die Haut sowie toxischer und mutagener Risiken in nahezu allen Anwendungsbereichen für Desinfektionsmittel auf dem Rückzug.

Die aliphatischen Alkohole Ethanol, Propanol-1 und Propanol-2 sind als Wirkstoffe zur Desinfektion von Haut und Händen beziehungsweise für die Haut- und Händeantiseptik seit langem bekannt. Mit Desinfektionsmitteln und Antiseptika auf der Basis von Alkoholen können bei kurzen Einwirkzeiten von 30 bis 60 Sekunden Keimzahlreduktionen von bis zu 99,9 % erzielt werden. Eine allgemeine, kurzgefasste Darstellung der mikrobiziden Wirksamkeit von Alkoholen findet sich in dem Buch: K.H. Wallhäußer, "Praxis der Sterilisation, Desinfektion und Konservierung", G. Thieme Verlag, Stuttgart, New York, 5. Auflage, S. 469 - 474*.*

Alkohole besitzen eine bakterizide Wirkung, die von Methanol zu Propanol zunimmt. Verwendet werden vor allem Ethanol, n-Propanol und Isopropanol, wobei der Alkoholgehalt der Zubereitungen im allgemeinen zwischen 50 und 80 % liegt. Der wesentliche Vorteil von Alkoholen ist, dass der Wirkungseintritt sehr rasch erfolgt. Nachteilig ist, dass sie nicht gegen Sporen wirksam sind und dass die Wirkung nach sehr kurzer Zeit endet, da Alkohole schnell verdunsten. Eine antivirale Wirksamkeit von Alkoholen wird zwar diskutiert, aber erst jenseits einer hohen Konzentrationsgrenze, welche bei Ethanol bei ca. 80 % vermutet wird.

Es hat sich in der Praxis gezeigt, dass alkoholische Desinfektionsmittel und Antiseptika Viren und Spuren von Bacillus- und Clostridienarten nicht oder nicht in hinreichendem Maße abzutöten vermögen. Zwar kann man die Sporenfreiheit von alkoholischen Lösungen durch Filtration erreichen, allerdings kann in der Praxis nicht vollständig ausgeschlossen werden, dass Keimsporen (nachträglich) in die Präparate gelangen, beispielsweise beim kurzzeitigen Öffnen der Aufbewahrungsgefäße oder beim Abfüllen der Mittel in Behälter, die bereits Sporen enthalten. Aus diesem Grund besteht bei der Verwendung von alkoholischen Haut- und Handantiseptika stets ein gewisses Risiko einer durch Sporen verursachten Infektion.

Antiseptika sind besonders geeignet zur Behandlung der Haut. Antiseptika zeigen eine sehr gute Wirksamkeit gegen Dermatophyten und zeichnen sich überraschenderweise insbesondere dadurch aus, dass sie eine gute Wirksamkeit gegen Viren haben.

Die Bestandteile der Antiseptika agieren in bezug auf ihre antimikrobiellen und antiviralen Eigenschaften synergistisch, also in signifikanter Weise überadditiv.

Vorteilhaft ist demgemäss auch die Verwendung einer Zubereitung aus

| | | |
|---|---|---|
| (a) | 42 - 47 Gew.-% | 1-Propanol |
| (b) | 22 - 27 Gew.-% | 2-Propanol |
| (c) | 4 - 6 Gew.-% | Ethanol |
| (d) | Mindestens 20 Gew.-% | Wasser |
| (e) | Höchstens 0,0001 Gew.-% | an Substanzen, welche unter Normalbedingen als Festkörper vorliegen |
| (f) | Keinen wirksamen Gehalt | an weiteren Substanzen, welche sich durch viruzide Eigenschaften auszeichnen |

als Antiseptikum, insbesondere die Verwendung zur Bekämpfung oder Inaktivierung des HIV-Virus oder des Hepatitis B-Virus.

Besonders geeignet als Antiseptikum ist wiederum Chlorhexidin, 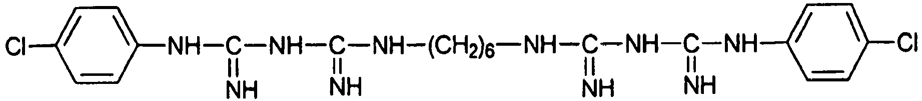 internationaler Freiname für 1,1'-Hexamethylenbis[5-(4-chlorphenyl)-biguanid], wobei als Antiseptikum das Dihydrochlorid, Diacetat und Digluconat verwendet werden.

Zur Anwendung als Pflaster werden die Gelmatrices als Schicht auf ein Trennmedium aus Papier, Folie o. ä. gepresst, gewalzt o. ä. und auf der Rückseite mit einem beliebigen Trägermaterial wie z.B. einer Polymerfolie, Textilien o.ä. kaschiert. Besonders bevorzugt werden die Gelmatrices im warmen Zustand mittels Dosierpumpe auf ein Trägermaterial aufgetragen und ganz besonders bevorzugt durch entsprechende Kavitäten in den Press- oder Walzwerken in einer dreidimensionalen Form ausgeführt. Die Form der erzeugten Pflaster wird durch die Form der Kavitäten bestimmt und unterliegt keiner Einschränkung, sie kann z.B. ellipsoid mit flach auslaufenden Rändern oder beispielsweise eckig ausgeführt sein.

Besonders vorteilhaft ist die Gelmatrix auf einer flexiblen Deckschicht aufgebracht, insbesondere bei der Verwendung als Pflaster. Aufgebaut ist ein entsprechendes Pflaster aus einem Träger wie Folien, Vliese, Gewebe, Schäume etc., der Klebmatrix und Abdeckfolie, Abdeckpapier oder Trennpapier zum Schutz der klebenden Matrix vor dem Gebrauch des Pflasters.

In einer weiteren bevorzugten Ausführungsform werden als Träger Polymerfolien, Vliese, Gewebe sowie deren Kombinationen eingesetzt. Als Trägermaterialien stehen u.a. Polymere wie Polyethylen, Polypropylen, Polyester, Polyether, Polyether-ester Copolymere und Polyurethan oder auch Naturfasern zur Auswahl.
Zusammenfassend kann festgehalten werden, dass als Trägermaterialien sich alle starren und elastischen Flächengebilde aus synthetischen und natürlichen Rohstoffen eignen. Bevorzugt sind Trägermaterialien, die so eingesetzt werden können, dass sie Eigenschaften eines funktionsgerechten Verbandes erfüllen. Beispielhaft sind Textilien wie Gewebe, Gewirke, Gelege, Vliese, Laminate, Netze, Folien, Schäume und Papiere aufgeführt. Weiter können diese Materialien vor- beziehungsweise nachbehandelt werden. Gängige Vorbehandlungen sind Corona und Hydrophobieren; geläufige Nachbehandlungen sind Kalandern, Tempern, Kaschieren, Stanzen und Eindecken.

Besonders vorteilhaft ist, wenn das Trägermaterial sterilisierbar, bevorzugt γ-(gamma) sterilisierbar, ist.

Ganz besonders bevorzugt sind Trägermaterialien mit einer guten Sauerstoff-, Luft- und Wasserdampfdurchlässigkeit, welche im Siebdruck oder analogen Verfahren punktuell mit der klebenden Polymermatrix versehen sind und an den Seitenrändern die aufgebrachte Gelmatrix nach außen überlappen. Dergestalt ausgefertigte erfindungsgemäße Matrices können an mechanisch stark beanspruchten Körperteilen wie Ellenbogen oder Kniegelenken selbstklebend fixiert werden, wo das eigene Haftvermögen der Hydrogele/Cataplasmen für eine dauerhafte Applikation nicht mehr genügt.

Die genannten Eigenschaften der Klebmatrix legen insbesondere die Verwendung für medizinische Produkte, insbesondere Pflaster, medizinische Fixierungen, Wundabdeckungen, orthopädische oder phlebologische Bandagen und Binden nahe.

Insbesondere die Verwendung der selbstklebenden Polymermatrix, bestehend aus einem Polyacrylsäurepolymer, Agar-Agar, Glycerin und Wasser, in Pflastern, Strips, Wundabdeckungen und/oder Bandagen ist eine äußerst einfache, hautverträgliche Möglichkeit der Wundversorgung bzw. Hautpflege. Die so ausgestatteten Pflaster, Wundabdeckungen oder Bandagen weisen eine individuell einstellbare Konsistenz und Klebkraft auf und sind gegenüber bekannten medizinischen Materialien äußerst wohlfeil. Die Polymermatrix kann alleine oder in Kombination mit geeigneten, beschichteten Trägermaterialien angewendet werden. Es können somit Produkte hergestellt werden, die auch an beweglichen Körperteilen, wie beispielsweise Finger oder Ellenbogen, angewendet werden können

Des weiteren ist die Verwendung der Polymermatrices, in die wasserlösliche oder hydrophobe Wirkstoffe eingearbeitet sind, als wirkstoffhaltige Pflastersysteme oder TTS zur gezielten Wirkstoffabgabe an die Haut besonders geeignet.
Die Verwendung der selbstklebenden Polymermatrix ist vor allem als wirkstoffhaltige Darreichungsform zur topischen oder buccalen Anwendung oder als Komponente eines, insbesondere monolithischen, TTS vorteilhaft anzusehen.

Beispielsweise kann durch den Einsatz von Menthol auf einem siebdruckbeschichteten Fließstoff ein Pflaster mit der Polymermatrix hergestellt werden, welches durch das Verdampfen, der Abgabe von Menthol und/oder Wasser, zu einem Kühleffekt bei kleinen Verbrennungen führt. Die Verwendung der selbstklebenden Polymermatrix enthaltend Menthol als Wirkstoff zur Anwendung bei Hautverbrennungen ist damit bevorzugt.

Werden als Wirkstoffe nichtsteroidale Antirheumatika eingesetzt bietet sich vorteilhafterweise die Polymermatrix zur Behandlung von Erkrankungen des rheumatischen Formenkreises an.

Ebenso erlaubt die Verwendung ätherischer Öle als Wirkstoffe die Anwendung der Polymermatrix bei Erkältungskrankheiten sowie zur Aromatherapie.

Die Matrix ist durch ihren einfachen Aufbau geeignet schnell, einfach und kostensparend Pflastersysteme oder TTS herzustellen. Durch die Möglichkeit der Einbindung von Wirkstoffen wird darüber hinaus die Anwendungsbreite solcher Pflastersystem stark erhöht und die Rentabilität bei der Herstellung erhöht.

Schließlich kann die Gelmatrix mit einem klebstoffabweisenden Trägermaterial, wie silikonisiertes Papier, eingedeckt oder mit einer Wundauflage oder einer Polsterung versehen werden. Auf seiner selbstklebend ausgerüsteten, später der Haut zugewandten Seite ist das erfindungsgemäße Pflaster über seine ganze Breite bis zum Gebrauch üblicherweise mit einem klebstoffabweisenden Trägermaterial abgedeckt. Dieses schützt die Selbstklebeschicht aus der gut hautverträglichen Klebemasse der Gelmatrix, die vorzugsweise im Transferverfahren aufgebracht worden ist, und stabilisiert zusätzlich das ganze Produkt. Die Abdeckung kann in bekannter Weise einstückig oder vorzugsweise zweiteilig ausgebildet sein.
Weitere Ausführungsformen können dergestalt sein, dass zwischen der Rückseite der Matrix und dem Abdeckträger sich eine zweite Matrix mit höherer Wirkstofflöslichkeit als Reservoir befindet. Dies könnte statt einer zweiten Matrix und Träger auch eine Tiefziehfolie mit reinem Wirkstoff sein.
Auf der Klebseite der Matrix befindet sich teilweise (z.B. am Rand) eine zweite Matrix mit hoher Klebkraft zur zusätzlichen Fixierung, aber ungenügender Wirkstofflöslichkeit.

Die wirkstofffreie Matrix befindet sich zwischen zwei nicht verankernden Folien und wird zur Fixierung genutzt. Die wirkstofffreie Matrix könnte auch (mit oder ohne Wundauflage) als Klebschicht für ein einfaches Wund-/Heftpflaster dienen.

Die Verwendung der Polymermatrix als medizinisches Pflastersystem, als Pflaster, Pad, Wundauflage oder Bandage ist besonders in flächiger Ausführungsform mit einer Gesamtfläche von 0,2 bis 1000 cm² geeignet. Damit werden beispielweise kleine (0,2 - 2 cm²) Verbrennungsbereiche der Haut abgedeckt oder großflächige Bereiche (bis zu 1000 cm²) zur Kühlung oder bei rheumatischen Beschwerden.
Bevorzugt ist dabei auch die Verwendung der selbstklebenden Polymermatrix in flächiger oder räumlicher Ausführungsform mit einem Polymermatrixgewichtsanteil von 0,1 bis 1000 g, insbesondere von 500 g. Die Form kann dabei rund, oval, eckig oder den Hautpartien angepasst gestaltet sein.

Die nachfolgenden Beispiele veranschaulichen die Gelmatrices. Die angegebenen Massenanteile beziehen sich auf die Gesamtmasse der Matrix.

### Beispiele I - III

| Bestandteil | **I** | **II** | **III** |
|---|---|---|---|
| Wasser | 69,90 % | 59,90 % | 49,90 % |
| Agar Agar | 2,00% | 2,00 % | 2,00 % |
| Glycerin | 20,00 % | 30,00 % | 40,00 % |
| Carbopol 980 | 8,00% | 8,00 % | 8,00% |
| NaOH | 0,10% | 0,10 % | 0,10 % |

Die Beispiele I - III zeigen bei konstantem Gehalt an Polyacrylsäure analoges Haftvermögen und bei ebenfalls konstantem Gehalt an Agar Agar aber steigendem Gehalt an Glycerin zunehmende Kohäsivität/Elastizität.

### Beispiele IV - VI

| Bestandteil | **IV** | **V** | **VI** |
|---|---|---|---|
| Wasser | 59,90 % | 55,90 % | 51,90 % |
| Agar Agar | 2,00 % | 2,00 % | 2,00 % |
| Glycerin | 30,00 % | 30,00 % | 30,00 % |
| Carbopol 980 | 8,00% | 12,00 % | 16,00 % |
| NaOH | 0,10 % | 0,10 % | 0,10 % |

Die Beispiele IV - VI zeigen bei konstantem Gehalt an Agar Agar und Glycerin analoge Kohäsivität/Elastizität und bei gleichzeitig steigendem Gehalt an Polyacrylsäure zunehmendes Haftvermögen.

### Beispiele VII - IX

| Bestandteil | **VII** | **VIII** | **IX** |
|---|---|---|---|
| Wasser | 52,1997 % | 56,1997 % | 58,2000 % |
| Sorbitol | 15,7000 % | 15,7000 % | 15,7000 % |
| Agar Agar | 2,0000 % | 2,0000 % | 2,0000 % |
| Glycerin | 15,0000 % | 15,0000 % | 15,0000 % |
| Carbopol 980 | 8,0000 % | 8,0000 % | 8,0000 % |
| NaOH | 0,1000% | 0,1000 % | 0,1000 % |
| Propandiol | 5,0000 % | ----- | ----- |
| FA Blau No. 1 | 0,0003 % | 0,0003 % | ----- |
| Menthol | 1,0000% | ----- | ----- |
| Dexpanthenol | 1,0000 % | ----- | ----- |
| Capsicum-Extrakt | ----- | 3,0000 % | ----- |
| Chlorhexidin-digluconat | ----- | ----- | 1,0000 % |

Die Formulierungen sind in jeglicher Hinsicht überaus befriedigende Präparate, die sich durch eine hervorragende Wirkung auszeichnen. Bei Anwendung der Matrices mit einem wirksamen Gehalt an erfindungsgemäß verwendeten Wirkstoffen ist eine wirksame Behandlung, aber auch eine Prophylaxe von entzündlichen Hautzuständen - auch dem atopischen Ekzem - und/oder der Hautschutz bei empfindlich determinierter trockener Haut möglich. Der erfindungsgemäße Wirkstoff bzw. die topische dermatologische Zubereitungen mit einem wirksamen Gehalt an erfindungsgemäßem Wirkstoff dient aber auch in überraschender Weise zur Beruhigung von empfindlicher oder gereizter Haut.

## Patentansprüche

1. Verwendung von einem in Wasser gelbildendem Polymer, Wasser, einem mit Alkohol nicht gelbildendem Meeresalgenextrakt und ein- oder mehrwertigem Alkohol zur Einstellung der Klebrigkeit, Kohesivität, Konsistenz und Elastizität von selbstklebenden Polymermatrices.

2. Verwendung nach Anspruch 1, **dadurch gekennzeichnet, dass** das in Wasser gelbildende Polymer aus mindestens einem Polyacrylsäurepolymer besteht.

3. Verwendung nach Anspruch 2, **dadurch gekennzeichnet, dass** als Polyacrylsäurepolymere Acrylat-Alkylacrylat-Copolymere mit folgender Struktur ausgewählt werden: 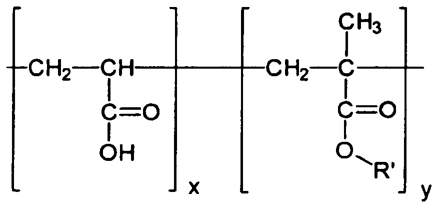 wobei R' ein Alkylrest und x bzw. y den jeweilige stöchiometrische Anteil der jeweiligen Comonomere darstellt.

4. Verwendung nach Anspruch 2 oder 3, **dadurch gekennzeichnet, dass** als Polyacrylsäurepolymere Copolymere aus C₁₀₋₃₀-Alkylacrylaten und einem oder mehreren Monomeren der Acrylsäure, der Methacrylsäure oder deren Ester, die kreuzvemetzt sind mit einem Allylether der Saccharose oder einem Allylether des Pentaerythrit, verwendet werden.

5. Verwendung nach einem der vorstehenden Ansprüche, **dadurch gekennzeichnet, dass** das in Wasser gelbildende Polymer in einer Menge von 2 - 55 Gew.%, bezogen auf die Gesamtmasse der Matrix, eingesetzt wird.

6. Verwendung nach einem der vorstehenden Ansprüche, **dadurch gekennzeichnet, dass** als Meeresalgenextrakt Agar-Agar und/oder Carrageenan gewählt wird.

7. Verwendung nach einem der vorstehenden Ansprüche, **dadurch gekennzeichnet, dass** der Meeresalgenextrakt in einer Menge von 0,1 - 15 Gew.%, bezogen auf die Gesamtmasse der Matrix, eingesetzt wird.

8. Verwendung nach einem der vorstehenden Ansprüche, **dadurch gekennzeichnet, dass** als ein- oder mehrwertige Alkohole Glycerin (1,2,3-Propantriol) eingesetzt wird.

9. Verwendung nach einem der vorstehenden Ansprüche, **dadurch gekennzeichnet, dass** der Alkohol in einer Menge von 1 - 85 Gew.%, bezogen auf die Gesamtmasse der Matrix, eingesetzt wird.

10. Verwendung nach einem der vorstehenden Ansprüche, **dadurch gekennzeichnet, dass** zusätzlich mindestens ein Wirkstoff enthalten ist.

11. Verwendung nach Anspruch 10, **dadurch gekennzeichnet, dass** der Wirkstoff gewählt wird aus der Gruppe Dexpanthenol, Capsaicin, Lidocain und/oder deren Salze, Menthol, Kampfer, Ibuprofen und/oder deren Salze wie -Lysinat, Ketoprofen, Eukalyptusöl, Pfefferminzöl, Chlorhexidin und/oder deren Salze, Silber oder Silberverbindungen, Jojobaöl, Aloe vera, Desinfektionsmittel und/oder Antiseptika.

## Claims

1. Use of a polymer which forms gel in water, water, a sea algae extract which does not form gel with alcohol, and monohydric or polyhydric alcohol, for adjusting the adhesiveness, cohesivity, consistency and elasticity of self-adhesive polymer matrices.

2. Use according to Claim 1, **characterized in that** the polymer which forms gel in water is composed of at least one polyacrylic acid polymer.

3. Use according to Claim 2, **characterized in that** polyacrylic acid polymers selected are acrylate-alkyl acrylate copolymers having the following structure: 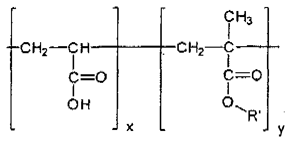 where R' is an alkyl radical and x and y respectively denote the respective stoichiometric fraction of the respective comonomers.

4. Use according to Claim 2 or 3, **characterized in that** polyacrylic acid polymers used are copolymers of C₁₀₋₃₀ alkyl acrylates and one or more monomers of acrylic acid, methacrylic acid or esters thereof which are crosslinked with an allyl ether of sucrose or with an allyl ether of pentaerythritol.

5. Use according to any one of the preceding claims, **characterized in that** the polymer which forms gel in water is used in an amount of 2% - 55% by weight, based on the total mass of the matrix.

6. Use according to any one of the preceding claims, **characterized in that** agar-agar and/or carrageenan are/is chosen as sea algae extract.

7. Use according to any one of the preceding claims, **characterized in that** the sea algae extract is used in an amount of 0.1% - 15% by weight, based on the total mass of the matrix.

8. Use according to any one of the preceding claims, **characterized in that** as monohydric or polyhydric alcohols glycerin (1,2,3-propanetriol) is used.

9. Use according to any one of the preceding claims, **characterized in that** the alcohol is used in an amount of 1% - 85% by weight, based on the total mass of the matrix.

10. Use according to any one of the preceding claims, **characterized in that** additionally at least one active substance is present.

11. Use according to Claim 10, **characterized in that** the active substance is chosen from the group consisting of dexpanthenol, capsaicin, lidocaine and/or salts thereof, menthol, camphor, ibuprofen and/or salts thereof such as lysinate, ketoprofen, eucalyptus oil, peppermint oil, chlorhexidine and/or salts thereof, silver or silver compounds, jojoba oil, aloe vera, disinfectants and/or antiseptics.

## Revendications

1. Utilisation d'un polymère formant un gel dans l'eau, d'eau, d'un extrait d'algues marines ne formant pas de gel avec un alcool et d'un alcool mono- ou polyhydrique pour l'ajustement de la pégosité, de la cohésivité, de la consistance et de l'élasticité de matrices polymères autoadhésives.

2. Utilisation selon la revendication 1, **caractérisée en ce que** le polymère formant un gel dans l'eau consiste en au moins un polymère poly(acide acrylique).

3. Utilisation selon la revendication 2, **caractérisée en ce qu'**on choisit comme polymères poly(acide acrylique) des copolymères acrylate-acrylate d'alkyle ayant la structure suivante : 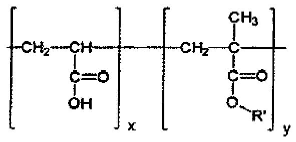 dans laquelle R' représente un radical alkyle et x ou, respectivement, y représente la proportion stoechiométrique respective des comonomères respectifs.

4. Utilisation selon la revendication 2 ou 3, **caractérisée en ce qu'**on utilise comme polymères poly(acide acrylique) des copolymères d'acrylates d'alkyle en C₁₀-C₃₀ et un ou plusieurs monomères de l'acide acrylique, de l'acide méthacrylique ou de leurs esters, qui sont réticulés avec un éther allylique du saccharose ou un éther allylique du pentaérythritol.

5. Utilisation selon l'une quelconque des revendications précédentes, **caractérisée en ce que** le polymère formant un gel dans l'eau est utilisé en une quantité de 2 - 55 % en poids, par rapport à la masse totale de la matrice.

6. Utilisation selon l'une quelconque des revendications précédentes, **caractérisée en ce qu'**on choisit comme extrait d'algues marines l'agar-agar et/ou le carraghénane.

7. Utilisation selon l'une quelconque des revendications précédentes, **caractérisée en ce qu'**on utilise l'extrait d'algues marines en une quantité de 0, 1 - 15 % en poids, par rapport à la masse totale de la matrice.

8. Utilisation selon l'une quelconque des revendications précédentes, **caractérisée en ce qu'**on utilise en tant qu'alcools mono- ou polyhydriques le glycérol (1,2,3-propanetriol).

9. Utilisation selon l'une quelconque des revendications précédentes, **caractérisée en ce qu'**on utilise l'alcool en une quantité de 1 - 85 % en poids, par rapport à la masse totale de la matrice.

10. Utilisation selon l'une quelconque des revendications précédentes, **caractérisée en ce qu'**en outre au moins une substance active est contenue.

11. Utilisation selon la revendication 10, **caractérisée en ce que** la substance active est choisie dans le groupe constitué par le dexpanthénol, la capsaïcine, la lidocaïne et/ou ses sels, le menthol, le camphre, l'ibuprofène et/ou ses sels tels que le lysinate d'ibuprofène, le kétoprofène, l'huile d'eucalyptus, l'huile de menthe poivrée, la chlorhexidine et/ou ses sels, l'argent ou des composés d'argent, l'huile de jojoba, l'Aloe vera, des désinfectants et/ou des antiseptiques.
